Europäisches Patentamt

European Patent Office

Office européen des brevets

(19)

(11) Publication number: **0 238 809 B1**

(12) # EUROPEAN PATENT SPECIFICATION

(45) Date of publication of patent specification: **20.02.91** (51) Int. Cl.⁵: **A61M 1/16**

(21) Application number: **87101232.4**

(22) Date of filing: **29.01.87**

(54) A detector system for the checking of a fluid tube connectable to a monitor.

(30) Priority: **24.03.86 SE 8601354**

(43) Date of publication of application:
**30.09.87 Bulletin 87/40**

(45) Publication of the grant of the patent:
**20.02.91 Bulletin 91/08**

(84) Designated Contracting States:
**BE CH DE ES FR GB IT LI NL**

(56) References cited:
**DE-A- 3 344 836**
**US-A- 3 812 482**
**US-A- 4 333 016**

**MEDICAL & BIOLOGICAL ENGINEERING &
COMPUTING, vol. 16, no. 3, May 1978, pages
278-284; J. BISERA: "Vascular interface system for automation of haemodynamic monitoring and therapy"**

(73) Proprietor: **GAMBRO AB**
**Post Box 10101**
**S-220 10 Lund(SE)**

(72) Inventor: **Ericson, Björn Inge**
**Utsättaregränd 93**
**S-22347 Lund(SE)**
Inventor: **Gummesson, Bengt-Ake Göran**
**Korgpilsgränd 4 Box 48**
**S-23040 Bara(SE)**
Inventor: **Örndal, Carl-Henry**
**Skomakaregatan 29**
**S-24100 Eslöv(SE)**

(74) Representative: **Boberg, Nils Gunnar Erik**
**Gambro AB Patent Department Box 10101**
**S-220 10 Lund(SE)**

EP 0 238 809 B1

## Description

### TECHNICAL FIELD

The present invention relates to a detector system for the checking of a fluid tube which is connectable to a monitor by means of which it is intended to control the flow within the tube, comprising a transmitter and receiver for a light beam of preferably infrared light and means for the conducting of this beam through the tube, when the same is assembled on the monitor, applied to a system for the extracorporeal treatment of blood, whereby the said tube may be filled with either blood or a priming fluid and the receiver is joined to an arrangement for comparing the values received with at least one reference value.

The detector system in accordance with the invention is intended in particular to be applied to a system for the extracorporeal treatment of blood, e g dialysis, the said tube being filled either with blood or a so-called priming fluid, that is to say a physiologically acceptable salt solution.

### BACKGROUND ART

A number of systems are known for the checking of fluid tubes assembled on guiding monitors. In dialysis monitors, for example, air detectors are known for checking whether any air is allowed to accompany the blood back to the patient. Furthermore, so-called blood-leakage monitors are known in dialysis monitors which sound an alarm if blood leaks over from the blood side to the dialysate. It is a disadvantage of these known systems that they are directly adapted to the actual treatment. As a result in certain cases they may cause more trouble than good, for example, during the running in or the so-called priming of a dialysis system. It has happened, for example, that during this running in blood leakage monitors gave false alarms because of air bubbles during the priming phase. Thus, an alarm was raised even though during this phase there clearly can be no kind of blood leakage. Furthermore, the afore-mentioned air-flow monitor naturally can sound an alarm during priming, if no special measures are adapted for by-passing the same.

It is the object of the present invention, therefore, to provide a special detector system of the above mentioned type and adapted especially to facilitate this priming phase. The invention may also be said to be an improvement of the system disclosed in US-A-3 812 482.

### DISCLOSURE OF INVENTION

To achieve the above mentioned object a detector system of the above mentioned type has been produced, which is characterized in that the said arrangement is programmed to establish:

a) whether the signal received exceeds a first higher reference value, corresponding to the absence of a tube,

b) whether the signal received exceeds a second lower reference value, corresponding to the assembled tube, which is empty or filled with a substantially colourless fluid,

c) whether the signal received falls short of the lower reference value, corresponding to the assembled tube being filled with blood.

Monitors of the type discussed here normally comprise one or more alarm functions intended to sound an alarm, if critical limit values are exceeded or fail to be attained in the extracorporeal treatment of blood. It is an advantage of the invention that certain of these alarm functions can be wholly disengaged or reduced in state a) and/or b), but are brought into full operation as soon as state c) occurs.

If the present invention is applied in connection with a monitor which comprises a clamp for the compression of the tube in connection with at least certain alarms, the further advantage is obtained that the said means for the conducting of the light beam through the tube can be assembled connected directly to the clamp in such a manner that the tube has to be installed in the claimp, when it is connected up in a state which can be sensed by the detector system.

If the invention is applied in connection with a monitor which comprises a blood pump intended to act on a pump segment incorporated in the tube, the further advantage is gained that the pump can be adapted so that it cannot be actuated in state a), whereas, on the other hand, it can be actuated a limited number of turns or during a limited time in state b) and without any hindrance in state c).

Monitors with transmitters and receivers of the above mentioned type normally have these and associated electrical components installed in the monitor itself or directly on its front. If this is the case, the above mentioned means for conducting the light beam advantageously can be included in a tube holder assembled on the monitor front through which the light is adapted to be conducted first substantially straight outwards from the front, subsequently, after it has passed the tube, to be refracted in the opposite direction towards the front. In this way the risk of the effects of external light interference is reduced, at the same time as this holder can be given a very simple technical design. It may be formed, for example, in one piece by die casting provided with a circular passage or guide for the tube, into which the latter can be inserted via a securing device which may be in the form of a slot which is narrower than the tube.

Preferably the signal sent out from the transmitter is given a defined form, e g the form of a pulse train of a defined frequency. Through this it becomes possible to compare the signal received by the receiver with that sent out in order to discover any interferences.

In a preferred embodiment of the system in accordance with the invention it comprises a main computer adapted so as to send out the said signals or beams via the transmitter and receiver to two comparators for comparison with a higher and lower reference signal respectively, which may also be issued from the computer or from a so-called safety computer. Appropriately a second computer, a so-called safety computer is provided for the checking and comparing of all the data read against given preset values, based e g on information fed into the main computer.

## BRIEF DESCRIPTION OF DRAWINGS

The invention is described in more detail in the following with reference to the attached drawings, wherein

Fig 1 shows schematically the most important outer components of a system in accordance with the invention,

Fig 2 a detector incorporated in the system, and

Fig 3 a schematic circuit diagram for the same.

## BEST MODE OF CARRYING OUT THE INVENTION

Although the invention is intended first and foremost to be used for the control of dialysis and will be described, therefore, in the following with reference to such an application, it will be obvious to those versed in the art, that the invention can be applied also, for example, to another extracorporeal blood treatment or to the control in general of different flows through a tube which can be connected to a monitor.

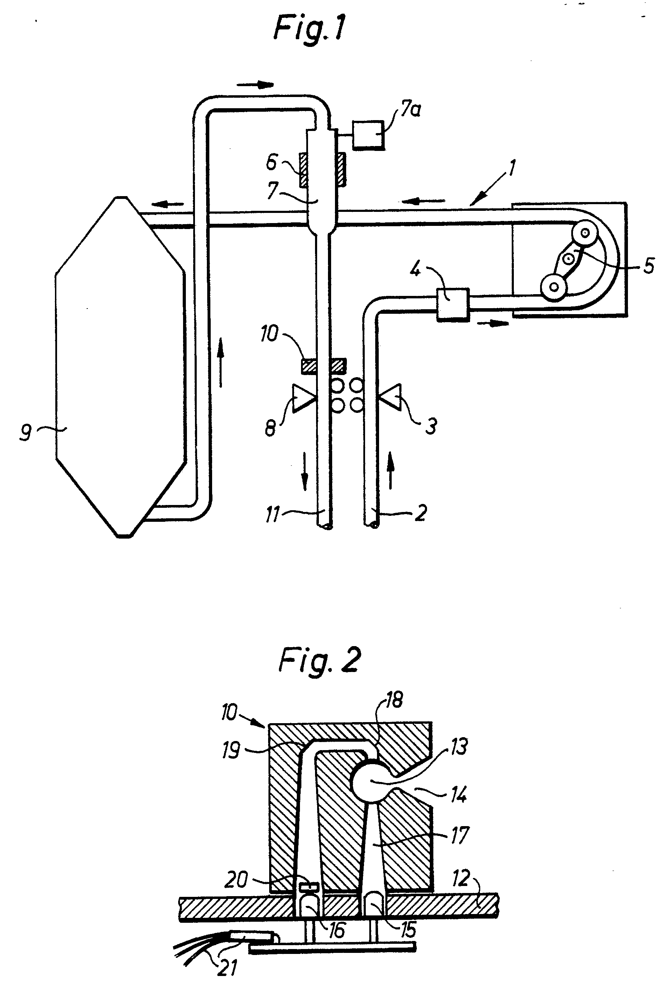

In Fig 1 is thus shown such a tube or tube system which as a whole is referred to by numeral 1 and whose one end 2 is intended for the withdrawal of blood from a patient. The tube or the tube system is normally connected to the front of a control monitor. For the sake of simplicity only certain components, taken from this front, are shown in Fig 1, namely an arterial clamp 3, with the help of which the flow of blood can be stopped, e g in connection with alarms, an arterial pressure controller 4 of conventional type, a pump 5, a combined air detector and holder 6 for a drip chamber 7 and a conventional venous clamp 8. In the Figure is shown schematically, moreover, dialyzer 9 and a detector 10 essential for the invention. The latter is arranged directly connected to the venous clamp 8 in such a manner that the tube can be assembled in this detector only when it is also assembled in the venous clamp 8. Numeral 11 designates the other end of the tube or tube set 1 which is intended to be connected to the patient for return of the blood after the dialysis. Finally 7a in Fig 1 designates a venous pressure gauge connected to the drip chamber 7. The arterial pressure controller 4 and the venous pressure gauge 7a appropriately are connected to different pressure gauges and to a computer included in the monitor in such a manner that an alarm is released when certain pressure limits are exceeded or fail to be reached.

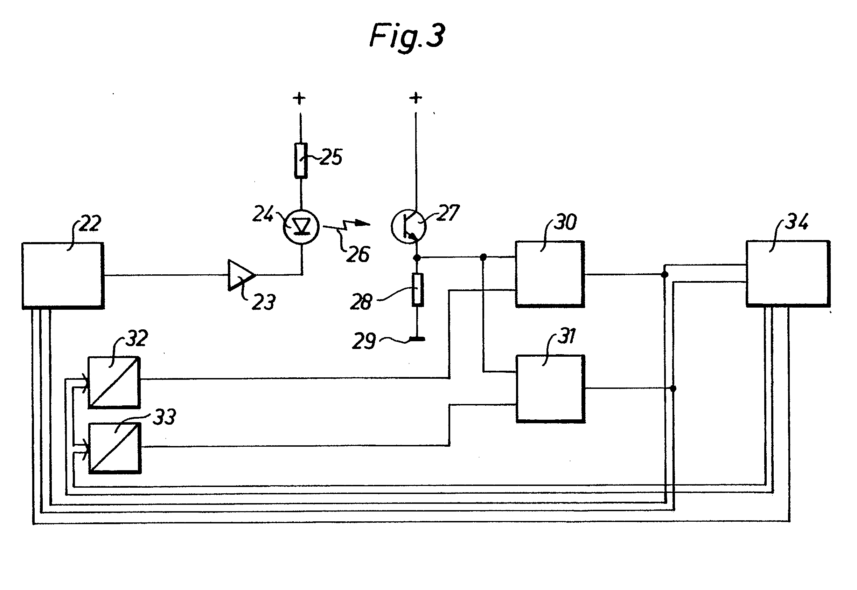

In Fig 2 is shown the detector 10 fitted on a monitor front 12. The actual tube holder is formed by a circular passage or guide 13 into which the tube can be inserted via a securing device 14 in the form of a slot which is narrower than the tube. In the monitor front is a transmitter 15 for preferably infrared light assembled together with a receiver 16 for the same light. This light is made to pass via a light conductor 17 through the circular passage 13 and thereby the tube 1, if the same is in assembled position. The light is then refracted with the help of angled surfaces 18 and 19, so that, passing through a wavelength filter 20, it is directed towards the receiver 16. Reference 21 finally designates how transmitter and receiver can be connected to the rest of the electronics inside the monitor. This electronics pack is described in more detail in Fig 3.

The electronics pack included may, of course, be designed in many different ways. In Fig 3 is shown, however, a preferred realization, wherein a main computer 22 is adapted to put out a signal via a driver stage, e g a transistor 23, to an infrared light-emitting diode 24 which via a resistor 25 is connected to a positive pole. From the light-emitting diode 24 is emitted a light beam 26 which is received by a phototransistor 27, which on one side is connected to a positive pole and on the other side via a resistor 28 to earth 29. The signal obtained is passed to two comparators 30 and 31, each of which obtains a reference signal from a so-called safety computer 34 via two converters 32 and 33. The latter convert a digital signal from the safety computer 34 to an analogue signal which can be compared with the analogue signal from the phototransistor 27. The upper converter 32 transmits a higher reference signal corresponding to a wholly disconnected tube, whereas the lower converter 33 sends out a lower signal corresponding to an assembled empty tube or an assembled tube filled with a substantially transparent fluid. By means of the comparators 30 and 31 it is thus possible to establish whether at all a tube is installed in the holder 10 and whether this tube is filled with blood or not. If blood is present in the tube the signal 26 is practically wholly extinguished, so that the value obtained from the phototransistor 27 will be located clearly below the reference signal from the converter 33. Values obtained from the comparators 30 and 31 are transmitted evidently not only to the main computer 22 but also to the above mentioned auxiliary or safety computer 34.

SUPPLEMENTARY INFORMATION

The plastics from which tubes of the above mentioned type normally are manufactured have essentially no absorption peaks within the range 850 nm to 1000 nm. Hence the wavelength of the infrared light is chosen appropriately within this range. It has been found particularly suitable to use a wavelength of 940 nm where these plastics have practically no absorption at all. The refractive index of these plastics is normally within the range 1.4-1.6 depending on the material and the wavelength of the light. This may be made use of for detecting an empty tube or a tube filled with a substantially transparent fluid. Owing to the tube being round it acts as a lens and refracts the light. This causes the intensity to diminish on the receiver side. In order to obtain the same measured value every time under the same conditions, the tube must not be deformed in the holder but should be "loose", whilst it must still be maintained in a certain place.

It is suitable for the detector to be calibrated before any tube is inserted. This is in order to compensate for any ripples within the light transmission system. If a tube is incorporated already in the holder 10 when the system is put into operation, the computer 22 may be adapted so that it starts out instead from the last calibration result.

The smallest opening within the light conductor 17 on the transmitter side ought to be selected according to the smallest tube dimension used. In practice it has been found appropriate to use a circular opening of a diameter of approx. 1.5 mm, a square opening of 1.5 mm side length or a gap of dimensions 1.5 mm × 5 mm. The corresponding smallest opening on the receiver side ought to be a little larger. It may, for example, have a corresponding diameter or side of 2 mm. In this manner good reliability is obtained with different tube diameters down to at least 1.5 mm. Such fine-calibre tubes, however, ought to be fixed in some manner in the light beam path.

Naturally the invention is not limited simply to the embodiment described above, but can be varied within the scope of the following claims. the components shown schematically, for example, may be designed in a different manner.

## Claims

1. A detector system for the checking of a fluid tube (1) which is connectable to a monitor (12), by means of which it is intended to control the flow within the tube, comprising a transmitter (15) and a receiver (16) respectively for a light beam of preferably infrared light, and means (17) for the con ducting of this beam through the tube when the same is assembled on the monitor, applied to a system for the extracorporeal treatment of blood, whereby the said tube (1) may be filled with either blood or a priming fluid and the receiver (16) is joined to an arrangement (30 or 31) for comparing the values received with at least one reference value, **characterized** in that the said arrangment is programmed to establish:

   a) whether the signal received exceeds a first higher reference value, corresponding to the absence of a tube,

   b) whether the signal received exceeds a second lower reference value, corresponding to the assembled tube, which is empty or filled with a substantially colourless fluid,

   c) whether the signal received falls short of the lower reference value, corresponding to the assembled tube being filled with blood.

2. A detector system in accordance with claim 1, the monitor (12) comprising one or more alarm functions intended to sound an alarm if certain critical limit values are exceeded or fail to be attained in the extracorporeal treatment of blood, **characterized** in that certain of these alarm functions are wholly disengaged or reduced in state a) and/or b), but are brought into full operation as soon as state c) occurs.

3. A detector system in accordance with anyone of the preceding claims, applied in connection with a monitor (12) which comprises a clamp (8) for compression of the tube (1) in connection with at least certain alarms, **characterized** in that the said means (17) for the conducting of the light beam through the tube (1) is assembled directly connected to the clamp (8) in such a manner that the tube (1) has to be installed in the clamp (8) when it is connected up in a state which can be sensed by the detector system.

4. A detector system in accordance with anyone of the preceding claims, the monitor (12) comprising a blood pump (5) intended to act on a pump segment incorporated in the tube, **characterized** in that the pump (5) is arranged so that it cannot be actuated in state a) whilst it can be actuated a limited number of turns or during a limited time in state b), and with out any hindrance in state c).

5. A detector system in accordance with anyone of the preceding claims, the said transmitter (15) and receiver (16) and associated electrical components (e g 24,27) being installed in the monitor (12) or directly on its front, **characterized** in that the said means (17) for conducting the light beam is in cluded in a tube holder (10) assembled on the monitor front, through which the light is intended to be condcuted first sub stantially straight outwards from the front, subsequently, after it has passed the tube, to be refracted in the opposite direc tion towards the front.

6. A detector system in accordance with claim 5, **charac terized** in that the tube holder (10) comprises a preferably circular passage and guide (13) for the tube (1), into which the latter can be inserted via a securing device, for example, in the form of a slot (14) which is narrower than the tube.

7. A detector system in accordance with anyone of the preceding claims, **characterized** in that the signal sent out from the transmitter (15) is of a defined form, e g the form of a pulse train of a defined frequency, and that the signal received by the receiver (16) is compared with that sent out in order to discover any interferences.

8. A detector system in accordance with anyone of the preceding claims, **characterized** in that a main computer (22) is adapted to send out signals or beams via the transmitter (15) and the receiver (16) to two comparators (30,31), for comparison with a higher or a lower reference signal respectively likewise issued from the computer (22) or from a so-called safety compu ter.

## Revendications

1. Système de détection pour le contrôle d'un tube de fluide (i) qui est connectable à un appareil de surveillance (12),au moyen duquel on prévoit de contrô ler l'écoulement dans le tube, comprenant un émetteur (15) et un récepteur (16) respectivement pour un faisceau lu mineux de préférence en lumière infrarouge, et des moyens (17) pour diriger ce faisceau à travers le tube lorsque celui-ci est monté sur l'appareil de surveillance, ap pliqué à un système pour le traitement extracorporel du sang, de sorte que ledit tube (1) peut être rempli de sang ou d'un fluide d'amorçage, et le récepteur (16) est relié à un dispositif (30 ou 3l)pour comparer les va leurs reçues avec au moins une valeur de référence, ca ractérisé en ce que le dit dispositif est programmé pour déterminer :

(a) si le signal reçu dépasse une première valeur de référence supérieure, ce qui correspond à l'absence d'un tube,

(b) si le signal dépasse une deuxième valeur de référence inférieure, ce qui correspond à un tube monté qui est vide ou rempli d'un fluide sensiblement incolore,

(c) si le signal reçu n'atteint pas la valeur de référence inférieure, ce qui correspond au tube monté et rempli de sang.

2. Système de détection suivant la revendi cation 1, l'appareil de surveillance (12) comprenant une ou plusieurs fonctions d'alarme prévues pour émettre une alarme si certaines valeurs limites critiques sont dépassées ou ne peuvent pas être atteintes dans le traitement extracorporel du sang, caractérisé en ce que certaines de ces fonctions d'alarme sont entièrement mises hors circuit ou réduites dans l'état (a) et/ou (b), mais sont mises en fonctionnement complet dès que l'état (c) se produit.

3. Système de détection suivant l'une quel conque des revendications précédentes appliqué en com binaison avec un appareil de surveillance (12) qui com prend un clamp (8) pour compression du tube (1) en relation avec au moins certaines alarmes, caractérisé en ce que lesdits moyens (17) pour diriger le faisceau lumineux à travers le tube (1) sont assemblés en liaison directe avec le clamp (8) d'une manière telle que le tube (1) doit être installé dans le clamp (8) lorsqu'il est raccordé dans un état qui peut être détecté par le système de détection.

4. Système de détection suivant l'une des revendications précédentes, l'appareil de surveillance (12) comprenant une pompe à sang (5) prévue pour agir sur un segment de pompe incorporé dans le tube, caracté risé en ce que la pompe (5) est agencée de sorte qu'elle ne peut pas être actionnée dans l'état (a), tandis qu'elle peut être actionnée d'un nombre limité de tours ou pendant un temps limité dans l'état (b) et sans aucune limitation dans l'état (c).

5. Système de détection suivant l'une quelconque des revendications précédentes, ledit émetteur (15) et ledit récepteur (16) et les composants électriques associés (par exemple 24,27) étant installés dans l'appareil de surveillance (12) ou directement sur sa façade, caractérisé en ce que lesdits moyens (17) pour diriger le faisceau lumineux sont inclus dans un porte tube (10) monté sur la façade de l'appareil de surveillan-ce et à travers lequel on prévoit de conduire la lumière d'abord sensiblement en ligne droite vers l'extérieur à partir de la façade, la lumière étant ensuite réfractée dans la direction opposée, vers la façade, après avoir traversé le tube.

6. Système de détection suivant la revendication 5, caractérisé en ce que le porte-tube (10) comprend un passage et un guidage de préférence circulaire (13) pour le tube (1), dans lequel ce dernier peut être inséré par l'intermédiaire d'un dispositif de fixation, par exemple sous la forme d'une fente (14) qui est plus étroite que le tube.

7. Système de détection suivant l' une quelconque des revendications précédentes, caractérisé en ce que le signal émis par l'émetteur (15) est d'une forme définie, par exemple la forme d'un train d'impulsion d'une fréquence définie, et en ce que le signal reçu par le récepteur (16) est comparé avec celui qui est émis, afin de déterminer des interférences éventuelles.

8. Système de détection suivant l'une quelconque des revendications précédentes, caractérisé en ce qu'un processeur principal (22) est prévu pour envoyer des signaux ou des faisceaux, par l'inter médiaire de l'émetteur (15) et du récepteur (l6), à deux comparateurs (30,31) pour comparaison avec un signal de référence supérieure ou un signal de référence inférieure respectivement,provenant également du processeur (22) ou d'un processeur dit de sécurité.

**Ansprüche**

1. Detektorsystem für die Überwachung eines Fluidrohres (1), welches an einen Monitor (12) anschließbar ist, mit Hilfe dessen der Fluß innerhalb des Rohres kontrolliert werden soll, mit einem Sender (15) bzw. einem Empfänger (16) für einen Lichtstrahl aus vorzugsweise Infrarotlicht und mit Einrichtungen (17) für das Leiten dieses Strahls durch das Rohr, wenn dasselbe an dem Monitor montiert ist, unter Anwendung auf ein System für die extrakorporale Behandlung von Blut, wobei das Rohr (1) entweder mit Blut oder mit einem Ansaugfluid gefüllt sein kann und der Empfänger (16) mit einer Anordnung (30 oder 31) zum Vergleichen der empfangenen Werte mit zumindest einem Referenzwert verknüpft ist, dadurch gekennzeichnet, daß die Anordnung so programmiert ist, daß sie feststellt:

a) ob das empfangene Signal einen ersten höheren Referenzwert übersteigt, der der Abwesenheit des Rohres entspricht,

b) ob das empfangene Signal einen zweiten unteren Referenzwert übersteigt, der dem montierten Rohr entspricht, welches leer oder mit einem im wesentlichen farblosen Fluid gefüllt ist,

c) ob das empfangene Signal unter den unteren Referenzwert fällt, was dem mit Blut gefüllten, installierten Rohr entspricht.

2. Detektorsystem nach Anspruch 1, bei welchem der Monitor (12) eine oder mehrere Alarmfunktionen aufweist, die einen Alarm ertönen lassen sollen, wenn bestimmte kritische Grenzwerte überschritten oder bei der extrakorporalen Behandlung von Blut nicht eingehalten werden, dadurch gekennzeichnet, daß bestimmte Alarmfunktionen in dem Zustand a) und/oder b) völlig außer Betrieb oder reduziert sind, jedoch voll in Betrieb gesetzt werden, sobald der Zustand c) auftritt.

3. Detektorsystem nach einem der vorstehenden Ansprüche, welches in Verbindung mit einem Monitor (12) angewendet wird, der eine Klemme (8) zum Zusammendrücken des Rohres (1) in Verbindung mit zumindest bestimmten Alarmen aufweist, dadurch gekennzeichnet, daß die Einrichtung (17) zum Leiten des Lichtstrahles durch das Rohr (1) in direktem Anschluß mit der Klemme (8) montiert ist, derart, daß das Rohr (1) in der Klemme (8) installiert sein muß, wenn es in einem Zustand angeschlossen ist, der von dem Detektorsystem erfaßt werden kann.

4. Detektorsystem nach einem der vorstehenden Ansprüche, wobei der Monitor (12) eine Blutpumpe (5) aufweist, die an einem Pumpabschnitt wirken soll, der in dem Rohr enthalten ist, dadurch gekennzeichnet, daß die Pumpe (5) so angeordnet ist, daß sie in dem Zustand a) nicht betätigt werden kann, während sie für eine begrenzte Anzahl von Drehungen oder während einer begrenzten Zeit in dem Zustand b) und ohne jede Beschränkung in dem Zustand c) betätigt werden kann.

5. Detektorsystem nach einem der vorstehenden Ansprüche, wobei der Sender (15) und der Empfänger (16) und die zugehörigen elektrischen Bauteile (z. B. 24,27) in dem Monitor (12) oder unmittelbar an seiner Vorderseite installiert sind, dadurch gekennzeichnet, daß die Einrichtung (17) zum Leiten des Licht strahles in einem Rohrhalter (10) aufgenommen ist, der an der Vorderseite des Monitors montiert ist, und durch welchen das Licht zunächst im wesentlichen gerade nach außen von der Vorderseite fortgeleitet werden soll und anschließend, nachdem es durch das Rohr hindurchgetreten ist, in entgegengesetzter Richtung zu der Vorderseite hin gebrochen werden soll.

6. Detektorsystem nach Anspruch 5, dadurch gekennzeichnet, daß der Rohrhalter (10) einen vorzugsweise kreisförmigen Durchgang und eine Führung (13) für das Rohr (1) aufweist, in welche das letztere über eine Befestigungsein richtung, beispielsweise in Form eines Schlitzes (14), der enger als das Rohr ist, eingesetzt werden kann.

7. Detektorsystem nach einem der vorstehenden Ansprüche, dadurch gekennzeichnet, daß das von dem Sender (15) ausgesandte Signal von einer definierten Form ist, beispielsweise der Form eines Pulszuges einer definierten Frequenz, und daß das von dem Empfänger (16) empfangene Signal mit dem ausgesandten Signal verglichen wird, um jegliche Interferenzen zu entdecken.

8. Detektorsystem nach einem der vorstehenden Ansprüche, dadurch gekennzeichnet, daß ein Hauptcomputer (22) so ausgelegt ist, daß er Signale oder Strahlen über den Sender (15) und den Empfänger (16) zu zwei Vergleichern (Komparatoren 30, 31) für den Vergleich mit einem höheren bzw. einem niedrigeren Referenzsignal aussendet, welches in ähnlicher Weise von dem Computer (22) oder von einem sogenannten Sicherheitscomputer ausgegeben wird.

## Fig.1

## Fig. 2

# Fig.3